(19) Europäisches Patentamt
European Patent Office
Office européen des brevets

(11) Veröffentlichungsnummer : **0 025 182**
**B1**

(12) **EUROPÄISCHE PATENTSCHRIFT**

(45) Veröffentlichungstag der Patentschrift : **26.10.83**

(51) Int. Cl.³ : **C 07 D319/20**, A 61 K 7/00, C 11 B 9/00

(21) Anmeldenummer : **80105080.8**

(22) Anmeldetag : **27.08.80**

(54) **3-Methyl-1,4-dioxa-bicyclo(4.4.0)decan-2-on, dessen Herstellung und Verwendung als Riechstoff sowie dieses enthaltende Riechstoffkompositionen.**

(30) Priorität : **05.09.79 DE 2935749**

(43) Veröffentlichungstag der Anmeldung : **18.03.81 Patentblatt 81/11**

(45) Bekanntmachung des Hinweises auf die Patenterteilung : **26.10.83 Patentblatt 83/43**

(84) Benannte Vertragsstaaten : **AT CH DE GB LI**

(56) Entgegenhaltungen : **US A 3 959 185**

(73) Patentinhaber : **Henkel Kommanditgesellschaft auf Aktien**
**Postfach 1100 Henkelstrasse 67**
**D-4000 Düsseldorf-Holthausen (DE)**

(72) Erfinder : **Schaper, Ulf-Armin, Dr.**
**Nixenstrasse 17**
**D-4000 Düsseldorf 13 (DE)**
Erfinder : **Bruns, Klaus, Dr.**
**Notburgaweg 6**
**D-4150 Krefeld-Traar (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 025 182 B1

# 3-Methyl-1,4-dioxa-bicyclo [4.4.0] decan-2-on, dessen Herstellung und Verwendung als Riechstoff sowie dieses enthaltende Riechstoffkompositionen

Es wurde gefunden, daß 3-Methyl-1,4-dioxa-bicyclo [4.4.0]-decan-2-on einen neuen Riechstoff mit einem interessanten Geruch nach Castoreum und außergewöhnlicher Haftfestigkeit darstellt.

Die Herstellung der erfindungsgemäßen neuen Verbindung kann nach zwei Herstellungsverfahren erfolgen. Als Ausgangsmaterial für die erste Synthese dienen Cyclohexenoxid oder 1,2-Cyclohexandiol. Diese werden mit Milchsäure oder α-Halogenpropionsäure oder deren Estern umgesetzt. Im allgemeinen wird dabei ein Gemisch von cis- und trans- 1,2-Cyclohexandiol mit D, L-Milchsäure bzw. racemischer α-Halogenpropionsäure oder deren Estern umgesetzt, so daß ein Gemisch von 4 Enantiomerenpaaren A bis D entsteht. Dieses Gemisch wird nicht getrennt, sondern als solches als Riechstoff verwendet. Die Umsetzung gemäß dieser ersten Synthese verläuft nach folgendem Schema :

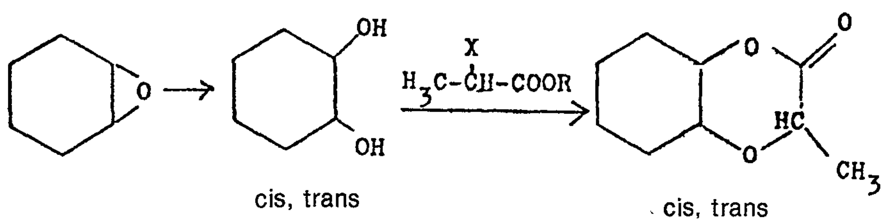

cis, trans　　　　cis, trans

X =OH, Cl, Br, J　R = H, niederes Alkyl

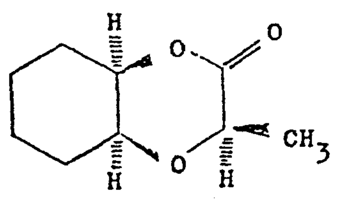

A

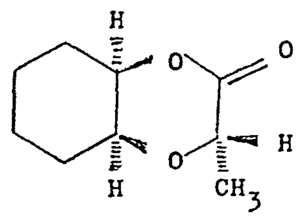

B

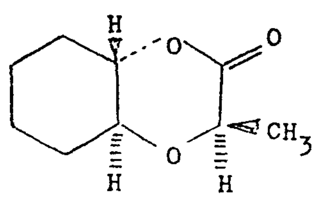

C

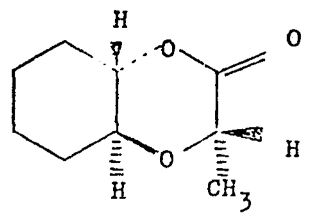

D

Am zweckmäßigsten wird bei dieser Synthese zuerst die Etherbindung hergestellt, indem man das Mononatriumsalz des 1,2-Cyclohexandiols mit 2-Chlorpropionsäureethylester bei 20-200 °C umsetzt. Anschließend wird durch saure Katalyse der Lactonring geschlossen :

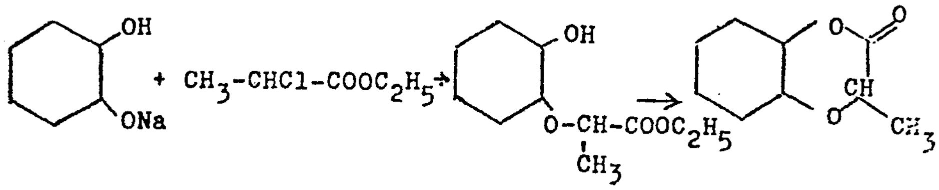

Eine zweite Synthesemöglichkeit für die erfindungsgemäße Verbindung besteht in einer Umlagerungsreaktion, die bei einer sauer katalysierten Umacetalisierung von Methylglyoxaldimethylacetal mit 1,2-Cyclohexandiol eintritt. Die Reaktion verläuft nach folgendem Schema :

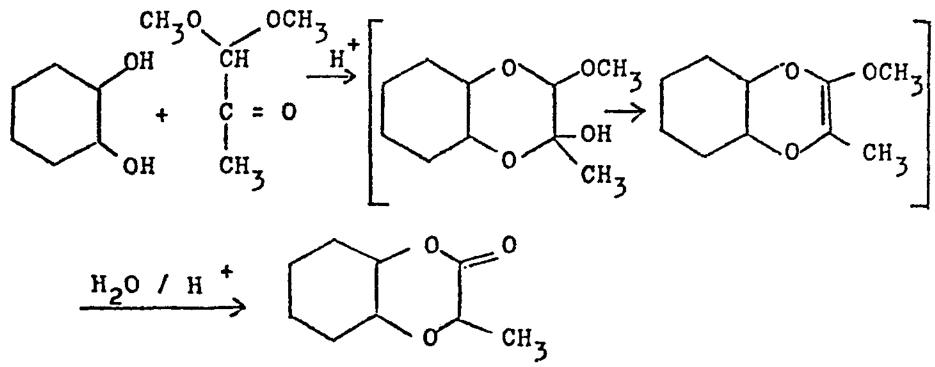


Das nach vorstehend aufgeführten Herstellungsverfahren erhaltene 3-Methyl-1,4-dioxa-bicyclo [4.4.0] decan-2-on zeichnet sich durch einen interessanten Geruch nach Castoreum sowie eine außergewöhnliche Haftfestigkeit aus. Ein weiterer Vorteil ist seine sehr gute Kombinationsfähigkeit zu neuartigen Kompositionen, denen es gleichfalls eine besondere Haftfestigkeit verleiht.

Der neue Riechstoff 3-Methyl-1,4-dioxa-bicyclo [4.4.0]-decan-2-on kann mit anderen Riechstoffen in den verschiedensten Mengenverhältnissen zu neuen Riechstoffkompositionen gemischt werden. Im allgemeinen wird sich sein Anteil in den Riechstoffkompositionen in den Mengen von 1 bis 50 Gewichtsprozent, bezogen auf die gesamte Komposition, bewegen. Derartige Kompositionen können zur Parfümierung von kosmetischen Präparaten wie Cremes, Lotionen, Duftwässern, Aerosolen, Toiletteseifen als auch in der Extrait-Parfümerie verwendet werden. Zur Parfümierung der verschiedenen Produkte werden diesen die Kompositionen im allgemeinen in Konzentrationen von 0,05 bis 2 Gewichtsprozent, bezogen auf das gesamte Produkt, zugesetzt.

Die nachfolgenden Beispiele sollen den Gegenstand der Erfindung näher erläutern.

## Beispiele

Herstellung von 3-Methyl-1,4-dioxa-bicyclo [4.4.0] decan-2-on

A) Durch Umsetzung von 1,2-Cyclohexandiol mit Chlorpropionsäureethylester

25,3 g (1,1 Mol) Natrium wurden unter trockenem Stickstoff in 500 ml Ethanol gelöst. Bei Raumtemperatur wurden zu der Lösung 116 g (1 Mol) 1,2-Cyclohexandiol gegeben. Danach wurde das Ethanol im Vakuum vollständig entfernt. Zu dem erhaltenen Mononatriumsalz des 1,2-Cyclohexandiols wurden bei 50 °C 163 g (1,2 Mol) 2-Chlor-propionsäureethylester gegeben und das Gemisch wurde 5 Stunden lang bei 80 °C zum Nachreagieren stehengelassen. Danach wurde das Reaktionsgemisch mit Wasser zersetzt und mit Schwefelsäure angesäuert. Die organische Phase wurde abgetrennt und die wäßrige Phase zweimal mit Ether extrahiert. Nach Vereinigung der organischen Phasen wurden diese mit Wasser und Bicarbonatlösung neutral gewaschen, über Natriumsulfat getrocknet und im Vakuum eingeengt. Das erhaltene Rohprodukt wurde im Hochvakuum destilliert. Es wurden 76 g an 3-Methyl-1,4-dioxa-bicyclo [4.4.0] decan-2-on mit folgenden Kennzahlen erhalten:

$Kp_{0,08\ mbar}$ = 78 bis 82 °C   Fp : 57 bis 62 °C

IR(Öl) $cm^{-1}$ : 2 985, 2 945, 2 870, 1 740, 1 450, 1 370, 1 330, 1 245, 1 235, 1 212, 1 130, 1 065, 855

NMR($CCl_4$) δ (ppm) : 1,48, d, J = 7 Hz($CH_3$) : 1,2-2, 2, m(Methylen) ; 3,4 m ; 4,1 m ; 4,5, q, J = 7 Hz (Methin).

Das Produkt besaß einen Geruch nach Castoreum.

B) Umacetalisierung von Methylglyoxaldimethylacetal

35 g (0,3 Mol) Methylglyoxaldimethylacetal und 23 g (0,2 Mol) 1,2-Cyclohexandiol wurden mit 1,5 g p-Toluolsulfonsäure 1/2 Stunde lang bei 90 bis 100 °C verrührt. Im Schwachen Vakuum wurde danach das gebildete Methanol im Verlauf von 3 Stunden abdestilliert und anschließend bei 20 mbar das überschüssige Glyoxalacetal entfernt. Der Rückstand wurde in Ether aufgenommen und mit Sodalösung bis pH 8 gewaschen. Nach dem Trocknen über Natriumsulfat wurde die Lösung eingeengt und im Vakuum destilliert. Es wurden 6,5 g an erfindungsgemäßen Produkt vom $Kp_{0,13\ mbar}$ 83 bis 86 °C erhalten.

## Beispiel 1

Riechstoffkomposition After-Shave Base

| | |
|---|---|
| 3-Methyl-1,4-dioxa-bicyclo [4.4.0] decan-2-on | 50 Gew.Teile |
| Boisambrene forte® (Henkel) | 200 Gew.Teile |

| | |
|---|---|
| Bergamottöl | 150 Gew.Teile |
| Linalylacetat | 100 Gew.Teile |
| Patchouliöl | 80 Gew.Teile |
| Cumarin | 60 Gew.Teile |
| Moschus Ambrette | 50 Gew.Teile |
| Eichenmoos Resin | 50 Gew.Teile |
| Lavandinöl | 50 Gew.Teile |
| Cistusöl (10 % DEP = Diethylphtalat) | 50 Gew.Teile |
| Vetiverylacetat | 40 Gew.Teile |
| Hydroxycitronellal | 40 Gew.Teile |
| Sandel® (H&R) | 40 Gew.Teile |
| Cedrylacetat | 30 Gew.Teile |
| Vanillin (50 % in DEP) | 10 Gew.Teile |
| | 1 000 Gew.Teile |

**Ansprüche**

1. 3-Methyl-1,4-dioxa-bicyclo [4.4.0] decan-2-on.

2. Verfahren zur Herstellung von 3-Methyl-1,4-dioxa-bicyclo [4.4.0] decan-2-on durch Umsetzung von Cyclohexenoxid beziehungsweise insbesondere 1,2-Cyclohexandiol mit Milchsäure oder α-Halogenpropionsäure oder deren Estern bei erhöhter Temperatur und Aufarbeitung des Reaktionsgemisches in üblicher Weise.

3. Verfahren zur Herstellung von 3-Methyl-1,4-dioxa-bicyclo [4.4.0] decan-2-on nach Anspruch 2 durch Umsetzung des Mononatriumsalzes des 1,2-Cyclohexandiols mit 2-Chlorpropionsäureethylester bei 20 bis 200 °C, Schließung des Lactonrings durch saure Katalyse und Aufarbeitung des Reaktionsproduktes in üblicher Weise.

4. Verfahren zur Herstellung von 3-Methyl-1,4-dioxa-bicyclo [4.4.0] decan-2-on durch sauer katalysierte Umacetalisierung von Methylglyoxaldimethylacetal mit 1,2-Cyclohexandiol bei erhöhter Temperatur und Aufarbeitung des Reaktionsproduktes in üblicher Weise.

5. Verwendung von 3-Methyl-1,4-dioxa-bicyclo [4.4.0] decan-2-on als Riechstoff.

6. Riechstoffkompositionen, dadurch gekennzeichnet, daß sie 3-Methyl-1,4-dioxa-bicyclo [4.4.0] decan-2-on in einer Menge von 1 bis 50 Gewichtsprozent, bezogen auf die gesamte Komposition, enthalten.

**Claims**

1. 4-methyl-2,5-dioxa-bicyclo [4.4.0] decan-3-one.

2. A process for the preparation of 4-methyl-2,5-dioxa-bicyclo [4.4.0] decan-3-one by reacting cyclohexene oxide or, more particularly, 1,2-cyclohexane diol with lactic acid or α-halogen propionic acid or esters thereof at elevated temperature and working up the reaction mixture in the usual way.

3. A process for the preparation of 4-methyl-2,5-dioxa-bicyclo [4.4.0] decan-3-one as claimed in Claim 2 by reacting the monosodium salt of 1,2-cyclohexane diol with 2-chloropropionic acid ethyl ester at 20 to 200 °C, closing the lactone ring by acid catalysis and working up the reaction product in the usual way.

4. A process for the preparation of 4-methyl-2,5-dioxa-bicyclo [4.4.0] decan-3-one by the acid-catalyzed trans-acetalization of methyl glyoxal dimethyl acetal with 1,2-cyclohexane diol at elevated temperature and working up of the reaction product in the usual way.

5. The use of 4-methyl-2,5-dioxa-bicyclo [4.4.0] decan-3-one as a fragrance.

6. Fragrance compositions, characterized in that they contain 4-methyl-2,5-dioxa-bicyclo [4.4.0] decan-3-one in a quantity of from 1 to 50 % by weight, based on the composition as a whole.

**Revendications**

1. 4-Méthyl-2,5-dioxa-bicyclo [4.4.0] décane-3-one.

2. Procédé de préparation de 4-méthyl-2,5-dioxa-bicyclo [4.4.0] décane-3-one par réaction d'oxyde de cyclohexène ou en particulier du 1,2-cyclohexanediol avec de l'acide lactique ou de l'acide α-halogénopropionique ou leurs esters à température élevée, et traitement du mélange de réaction de manière usuelle.

3. Procédé de préparation de 4-méthyl-2,5-dioxa-bicyclo [4.4.0] décane-3-one suivant la revendication 2, par réaction du sel monosodique du 1,2-cyclohexanediol avec le 2-chloropropionate d'éthyle à 20-200 °C, fermeture du cycle lactonique par catalyse acide et traitement du produit de réaction de manière usuelle.

4. Procédé de préparation de 4-méthyl-2,5-dioxa-bicyclo [4.4.0] décane-3-one par réaction d'acétalisation catalysée du méthylglyoxal-diméthylacétal avec le 1,2-cyclohexanediol à température élevée et traitement du produit de réaction de manière usuelle.

5. Utilisation de 4-méthyl-2,5-dioxa-bicyclo [4.4.0] décane-3-one comme parfum.

6. Compositions de parfum, caractérisées en ce qu'elles contiennent du 4-méthyl-2,5-dioxa-bicyclo [4.4.0] décane-3-one en une quantité de 1 à 50 % en poids par rapport à l'ensemble de la composition.